# EUROPEAN PATENT SPECIFICATION

(11) **EP 4 280 978 B1**
(45) Date of publication and mention of the grant of the patent: **29.04.2026**
(21) Application number: 22704800.6
(22) Date of filing: 21.01.2022
(51) Int. Cl.: A61B 17/11

(54) **DEVICE FOR THE TREATMENT OF ESOPHAGEAL ATRESIAS AND STENOSIS**
VORRICHTUNG ZUR BEHANDLUNG VON SPEISERÖHRENATRIEN UND STENOSE
DISPOSITIF POUR LE TRAITEMENT DE L'ATRÉSIE D'OESOPHAGE ET DE LA STÉNOSE

(30) Priority: 22.01.2021 IT 202100001184
(43) Date of publication of application: 29.11.2023
(73) Proprietor: SIDAM S.r.l., 41037 Mirandola (MO) (IT); Ospedale Pediatrico Bambino Gesù, 00165 Roma (IT)
(72) Inventor: AZZOLINI, Graziano, 41037 Mirandola (MO) (IT); FEDERICI DI ABRIOLA, Giovanni, 00165 Roma (IT); DALL'OGLIO, Luigi, 00165 Roma (IT)
(74) Representative: Brunacci, Marco
(86) International application number: PCT/IB2022/050547
(87) International publication number: WO 2022/157710

(56) References cited:
- EP-A1- 0 302 001
- CN-A- 108 606 930
- US-A1- 2005 228 412
- ANONYMOUS: "Pediatric Esophageal Atresia Device", 11 May 2017 (2017-05-11), pages 1 - 9, XP055846512, Retrieved from the Internet <URL:https://www.fda.gov/media/142080/download> [retrieved on 20211001]

## Description

### Technical Field

The present invention relates to a device for the treatment of esophageal atresias and stenosis.

### Background Art

As is well known, the esophagus is the first section of the digestive tract which allows food to pass from the mouth to the stomach.

Esophageal atresia consists of an interruption of the esophagus, which is then divided into two stumps, thus preventing the passage of food.

**In** addition, this malformation is often associated with the abnormal presence of communication with the trachea, which is responsible for allowing air to pass into the lungs.

The frequency with which esophageal atresia occurs is one infant per three thousand live births.

In the majority of cases, this malformation can be solved by direct anastomosis. However, in 10% of cases, the distance between the two stumps of the esophagus is such that it cannot be repaired by anastomosis (Long-Gap esophageal atresia).

Currently, multiple strategies are in use allowing a direct anastomosis to be carried out, thus avoiding the replacement of the esophagus with other viscera, such as e.g. the stomach, jejunum, or colon.

However, the direct esophagus-esophageal anastomosis is burdened by many drawbacks, comprising the risk of partial or total collapse of the anastomosis itself to which is added the formation of scarring stenosis.

These risks are directly proportional to the traction to which the anastomosis is subjected, as a result of the distance between the two esophageal stumps.

Although scarring stenosis can be successfully treated by means of endoscopic dilatations or esophageal stents, however, in cases of recurrent or treatment-resistant stenosis, resection of the stenotic tract is necessary to ensure adequate passage of the food bolus and nutrition of the patient without resorting to lifelong gastrostomy nutrition.

**In** addition, even partial collapse of the anastomosis, due to stretch between the two stumps, carries serious risks during the patient's life because of the possible onset of a dreaded mediastinitis, to which are added the risks of the formation of scarring stenosis of the anastomosis or the formation of tracheo-esophageal fistulas.

**In** order to reduce the stretch between the two ends of the esophagus, which is the main cause of the increased risk of collapse of the anastomosis, various surgical strategies have been devised adapted to lengthen the two esophageal stumps.

In fact, the main reason for the collapse of the anastomosis is represented by the fact that the stretch existing between the two stumps is inevitably discharged on the suture line which, even if carried out in a workmanlike manner, is subject to the risk of collapse due to decubitus of the stitches on the esophageal tissue.

Patients with this complication require prolonged ICU stay, prolonged parenteral or enteral nutrition and prolonged antibiotic and antifungal therapies.

In the most severe cases, new surgeries are forced on tissues which have already undergone surgery and therefore with greater intra- and post-operative risks. In some cases the prolonged use of the extracorporeal membrane oxygenation (ECMO) and repeated thoracotomies or sternotomies are required.

**In** addition, patients with scarring stenosis on the esophagus, as a result of previous esophageal anastomosis or caustic ingestion, may solve the stenosis conservatively through endoscopic dilation or placement of esophageal stents.

Such esophageal stents are described in patent document no. EP3151899.

**In** particularly severe cases with recurrent or treatment-resistant stenosis, resection of the stenotic tract remains the only possible strategy.

**In** addition, reconstruction of the continuity of the esophageal lumen is currently possible only in cases where the tract to be resected is not longer than 2-4 cm.

However, in some cases esophageal replacement remains the only option; even this solution is not without its drawbacks.

The replacement of the esophagus with other viscera is, in fact, burdened by a series of post-operative complications and problems over time caused by the fact that the viscera replacing the esophagus are not adequate to mimic the functioning of the esophagus itself.

Therefore, these are strategies to be limited for cases in which direct anastomosis is not possible or for complications of the same.

Even in the latter case, patients whose esophagus is replaced by jejunum or colon will, over time, experience dilation of the transposed viscera, particularly the colon, resulting in delayed emptying, mediastinal bulk, halitosis and pulmonary aspiration.

Similar disorders are also attributable to patients in whom the stomach is transposed in the chest (gastric pull-up); in detail, the latter suffer from gastro-oesophageal reflux and respiratory symptoms.

**In** addition, patients with esophageal replacement have in pediatric and adult age the persistence of the disorders described above, with the need for multiple hospitalizations for medical therapy and further surgery and consequent poor quality of life.

In light of these issues, there is currently a strong need to develop devices for the treatment of esophageal atresias and stenoses which allow replacing and/or reconstructing the missing esophageal tract thus avoiding the above mentioned drawbacks.

Other devices for the treatment of esophageal atresias and stenoses are know from US 2005/228412 A1, "Pedriatic Esophageal Atresia Device", CN 108 606 930 A and EP 0 302 001 A1.

### Description of the Invention

The main aim of the present invention is to devise a device for the treatment of esophageal atresias and stenoses which allows increasing the tensile strength of the anastomosis by means of the distribution of the existing stretch between the two stumps.

Within this aim, one object of the present invention is to devise a device for the treatment of esophageal atresias and stenoses which allows the two stumps to be gradually brought closer together while avoiding the risk of collapse, not only along the suture line of the same, but also along the entire length of the esophagus.

Another object of the present invention is to devise a device for the treatment of esophageal atresias and stenosis which allows the mentioned drawbacks of the prior art to be overcome within a simple, rational, easy and effective to use as well as affordable solution.

The aforementioned objects are achieved by the present device for the treatment of esophageal atresias and stenosis having the characteristics of claim 1.

The aforementioned objects are achieved by the present kit for the treatment of esophageal atresias and stenosis having the characteristics of claim 14.

### Brief Description of the Drawings

Other characteristics and advantages of the present invention will become more apparent from the description of a preferred, but not exclusive, embodiment of a device for the treatment of esophageal atresias and stenosis, illustrated by way of an indicative, yet non-limiting example, in the accompanying tables of drawings wherein:
Figure 1 is a front view of the device according to the invention;
Figure 2 is a schematic representation of the device according to the invention in use.

### Embodiments of the Invention

**In** a first aspect, the present invention relates to a device for the treatment of esophageal atresias and stenosis.

The device 1 comprises a tubular body 2 of elongated shape intended, in use, to be inserted inside an esophagus 3 affected by esophageal atresia and divided into a distal stump 4 and into a proximal stump 5.

It is specified that esophageal atresia is a pathology which causes the interruption of this section of the digestive tract, which, as a rule, allows the passage of food from the mouth to the stomach.

This interruption determines the formation of two stumps, i.e. two blind ducts spaced apart from each other, one placed above and one placed below.

It is specified that within the scope of the present disclosure, the adjectives "distal" and "proximal" are considered with reference to the operator employing the device 1.

Additionally, it is specified that in the context of the present disclosure, the adjectives "upper" (above), "lower" (below) refer to the condition in which the patient is standing vertically.

The tubular body 2, in particular, extends along a longitudinal direction and is made of a material flexible enough to allow it to be introduced into the patient via the oral route or through gastrostomy.

**In** the embodiments depicted in the figures, the tubular body 2 has a substantially circular cross-section and is internally hollow, defining an internal duct 6.

According to the invention, the device 1 comprises a magnetic element 7 associated with one end of the tubular body 2 and intended to be placed at the point where one of either the proximal stump 5 or the distal stump 4 is located.

The magnetic element 7 is of the type of a magnetized body adapted to attract a magnet of opposite polarity or a ferromagnetic material.

Preferably, the magnetic element 7 is made at least party of neodymium.

In addition, the magnetic element 7 has an elongated conformation.

Furthermore, the magnetic element 7 comprises at least one invitation countersink 8 made on an end portion thereof and adapted to facilitate the introduction of a guidance body 9.

According to the embodiment shown in the figures, the magnetic element 7 comprises two invitation countersinks 8 made on the end portions of the magnetic element 7, respectively.

Advantageously, the magnetic element 7 has a cylindrical conformation.

The guidance body 9 has an elongated conformation and is accommodated in the tubular body 2 and associated, at one end, with the magnetic element 7.

Preferably, the guidance body 9 comprises stiffening means 10 extending internally to the guidance body itself.

The stiffening means 10 are helically wound and are adapted to prevent the guidance body 9 from crushing as a result of movements of the tubular body 2.

**In** detail, the stiffening means 10 are made of elastomeric material with a predefined hardness.

Preferably, the aforementioned stiffening means 10 are made of a metallic material.

Furthermore, the device 1 comprises a containment casing 11 of the magnetic element 7 associated with the tubular body 2 by interposition of joining means 12.

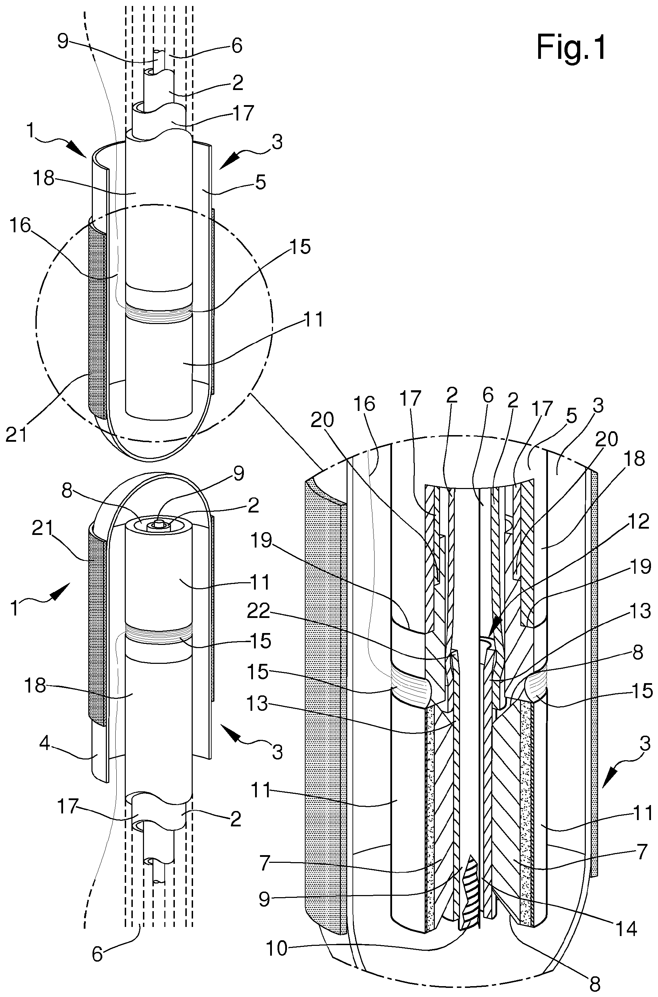

As visible in Figure 1, the magnetic element 7 is housed inside the containment casing 11 and protrudes inferiorly overhanging with respect to the latter.

This means that the magnetic element 7 protrudes inferiorly from the containment casing 11 towards the wall of the respective stump 4, 5.

In order to ensure the locking between the containment casing 11 and the magnetic element 7, the latter is associated with the containment casing itself by interposition of a layer of adhesive material of the type of a cyanoacrylate glue.

According to the invention the joining means 12 comprise a tubular duct 13 inserted inside a through channel 14 made on the magnetic element 7.

The tubular duct 13 is protruding cantilevered from the magnetic element 7 and coupled to the tubular body 2 by interlocking.

In detail, the tubular duct 13 protrudes above with respect to the containment casing 11.

Preferably, the tubular duct 13 is fitted to size inside the through channel 14.

In this regard, the tubular duct 13 has a flared extremity 22 configured to be coupled to the tubular body 2 by interlocking.

In detail, the end of the tubular body 2 undergoes a slight deformation due to the insertion by interference of the tubular duct 13 into the tubular body 2; this deformation is necessary to avoid the accidental detachment of the tubular body 2 from the tubular duct 13 due to the external stresses to which the tubular body 2 is subjected.

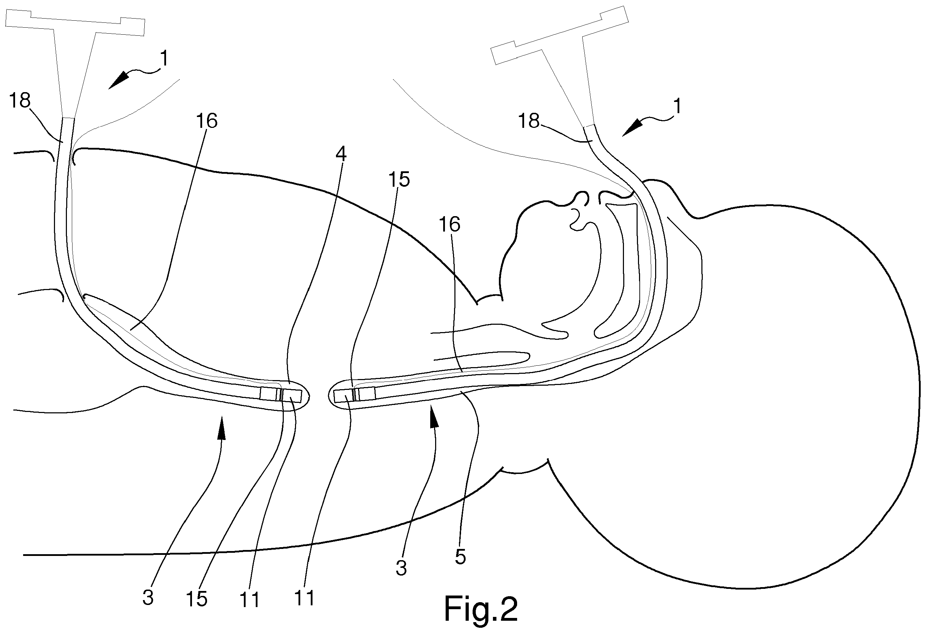

As can be seen in Figures 1 and 2, the containment casing 11 comprises at least one annular groove 15 adapted to house at least one suture thread 16 wrapped around the containment casing 11.

The suture thread 16 is adapted to allow removal of the containment casing 11 and, therefore, of the magnetic element 7 from the esophagus 3 of the patient.

**In** order to allow the positioning of the device 1 inside the esophagus 3 and the release of the containment casing 11 locked together with the magnetic element 7 at the point where the respective stump 4, 5 is located, the device 1 comprises pushing means 17 and ejection means 18.

**In** detail, the pushing means 17 are associated with the containment casing 11 and are configured to allow the magnetic element 7 to slide inside the esophagus 3.

**In** parallel, the ejection means 18 are associated with the containment casing 11 and configured to allow the release of the magnetic element 7 inside the esophagus 3.

The pushing means 17 comprise one pusher tube and the ejection means 18 comprise one ejector tube, the pusher tube 17 and the ejector tube 18 being concentric and associated with each other in a sliding manner.

**In** detail, the ejector tube 18 is arranged around the pusher tube 17 which, in turn, is arranged around the tubular body 2.

Preferably, at least one of either the pusher tube 17 or the ejector tube 18 is coupled by interlocking in a removable manner to the containment casing 11 and the other of either the pusher tube 17 or the ejector tube 18 abuts against the containment casing 11.

Advantageously, the containment casing 11 is provided with two abutment surfaces 19,20 made on the apical portion thereof and configured to abut against the pusher tube 17 and the ejector tube 18, respectively.

With reference to a preferred embodiment shown in the figures, the ejector tube 18 is coupled by interlocking in a removable manner to the containment casing 11 and the pusher tube 17 abuts against the containment casing 11 and is positioned between the ejector tube 18 and the tubular body 2; the pusher tube 17, due to an external force exerted thereon, pushes the containment casing 11 away and releases the coupling of the containment casing 11 from the ejector tube 18.

**In** detail, the abutment surfaces 19, 20 can be subdivided into a first abutment surface 19 and a second abutment surface 20.

The first abutment surface 19 is adapted to couple by interlocking to the ejector tube 18 and the second abutment surface 20 is adapted to abut against the pusher tube 17.

In detail, the ejector tube 18 is coupled by interference to the first abutment surface 19 and the pusher tube 17 is coupled to size to the second abutment surface 20.

The pusher tube 17, being positioned between the tubular body 2 and the ejector tube 18, is arranged between them in such a way that, during the movement of the device 1, it remains in that position and, after positioning, it allows the release thereof due to the force exerted by the pusher tube 17 on the second abutment surface 20. In other words, the pusher tube 17 and the ejector tube 18 are mutually movable in an axial manner so as to allow the pushing and positioning of the magnetic element 7.

In a second aspect, the present invention relates to a kit for the treatment of esophageal atresias and stenosis.

Such kit comprises:
- a device 1 arranged at the point where one of either the distal stump 4 or the proximal stump 5 is located;
- an auxiliary device comprising a ferromagnetic element, arranged at the point where the other of either the distal stump 4 or the proximal stump 5 is located and configured to interact by electromagnetic attraction with the device 1 so as to allow the distal stump 4 and the proximal stump 5 to approach each other.

According to a preferred embodiment, the kit comprises two devices 1 wherein the magnetic elements 7 have opposite polarity.

In addition, the kit comprises two reinforcing membranes 21 associable with the outer wall of the esophagus 3 at the point where the proximal stump 5 and the distal stump 4 are located, respectively.

Preferably, the aforementioned reinforcing membranes 21 are made at least partly of a biocompatible and absorbable material.

The device of the present invention is suitable to be used as follows.

The device 1 is introduced into the proximal stump 5 through the patient's mouth.

**In** parallel, the other device 1 is introduced into the distal stump 4 through a gastrostomy made on the patient's abdominal wall.

In detail, each device 1 is pushed to the bottom wall of the respective stump 4, 5.

At this point, the operator maneuvers the guidance body 9 so that the magnetic elements 7 are aligned and coaxial with each other.

After the magnetic elements 7 have been positioned, the operator exerts a pushing force on the pusher tube 17 in such a way as to allow the establishment of an attraction between the magnetic elements themselves and, as a result of an additional pushing exerted on the ejector tube 18, to allow decoupling between the ejector tube itself and the containment casing 11.

This allows releasing the containment casing 11 which remains positioned at the point where the respective stump 4, 5 is located, locked together with the tubular body 2.

The tubular body 2 exits the nose and/or gastrostomy of the patient.

The suture thread 16 also exits the nose and/or gastrostomy of the patient in such a way that, depending on specific clinical conditions, it can be hooked onto a tool or grasped and, by means of traction, allow removal of the magnetic element 7, i.e., of the containment casing 11 of the magnetic element itself.

It has in practice been ascertained that the described invention achieves the intended objects.

It is important to underline the fact that the particular expedient of providing the synergic combination of a magnetic element positioned at the point where a stump of the oesophagus and of another magnetic element of opposite polarity are located and positioned at the point where the opposite trunk allows the gradual, slow and mutual approach of the same, thus ensuring an anastomosis resistant to traction and avoiding an abrupt yielding of the oesophageal wall.

Substantially, the magnetic elements allow the two stumps to be brought close together and juxtaposed, thus avoiding the damage to the esophageal wall due to the traction force exerted on the latter.

In addition, the membrane prevents the elastic, tensile reaction of the esophageal wall, ensuring the creation of a tensile-resistant anastomosis.

## Claims

1. Device (1) for the treatment of esophageal atresias and stenosis, comprising at least one tubular body (2) of elongated shape intended, in use, to be inserted inside an esophagus (3) affected by esophageal atresia and divided into a distal stump (4) and into a proximal stump (5), the device comprising at least one magnetic element (7) associated with one end of said tubular body (2) and intended to be placed at the point where either said distal stump (4) or said proximal stump (5) is located **characterized by** a joining means (12) comprising at least one tubular duct (13) inserted inside a through channel (14) made on said magnetic element (7), said tubular duct (13) being protruding cantilevered from said magnetic element (7) and coupled by interlocking to said tubular body (2).

2. Device (1) according to claim 1, **characterized by** the fact that it comprises at least one containment casing (11) of said magnetic element (7) associated with said tubular body (2) by interposition of the joining means (12).

3. Device (1) according to claim 1, **characterized by** the fact that said tubular duct (13) has a flared extremity (22) configured to be coupled by interlocking to said tubular body (2).

4. Device (1) according to claim 2, **characterized by** the fact that said containment casing (11) comprises at least one annular groove (15) adapted to house at least one suture thread (16) wrapped around said containment casing (11).

5. Device (1) according to claim 2 or 4, **characterized by** the fact that it comprises pushing means (17) associated with said containment casing (11) and configured to allow said magnetic element (7) to slide inside said esophagus (3).

6. Device (1) according to claim 2, 4 or 5, **characterized by** the fact that it comprises ejection means (18) associated with said containment casing (11) and configured to allow the release of said magnetic element (7) inside said esophagus (3).

7. Device (1) according claim 5 or claim 6 when depending on claim 5, **characterized by** the fact that said pushing means (17) comprise at least one pusher tube (17) and said ejection means (18) comprise at least one ejector tube (18), said pusher tube (17) and said ejector tube (18) being concentric and associated with each other in a sliding manner.

8. Device (1) according to claim 7, **characterized by** the fact that at least one of either said pusher tube (17) or said ejector tube (18) is coupled by interlocking in a removable manner to said containment casing (11) and the other of either said pusher tube (17) or said ejector tube (18) abuts against said containment casing (11).

9. Device (1) according to claim 7 or 8, **characterized by** the fact that:
- said ejector tube (18) is coupled by interlocking in a removable manner to said containment casing (11);
- said pusher tube (17) abuts against said containment casing (11) and is positioned between said ejector tube (18) and said tubular body (2);
said pusher tube (17), due to an external force exerted thereon, pushes said containment casing (11) away and releases the coupling of said containment casing (11) from said ejector tube (18).

10. Device (1) according to one or more of the preceding claims, **characterized by** the fact that said magnetic element (7) is made at least partly of neodymium.

11. Device (1) according to one or more of the preceding claims, **characterized by** the fact that it comprises at least one guidance body (9) accommodated in said tubular body (2) and associated, at one end, with said magnetic element (7).

12. Device (1) according to one or more of the preceding claims, **characterized by** the fact that said guidance body (9) comprises stiffening means (10) extending internally to said guidance body itself.

13. Kit for the treatment of esophageal atresias and stenosis, **characterized by** the fact that it comprises:
- at least one device (1) according to one or more of the preceding claims suitable to be arranged at the point where one of either said distal stump (4) or said proximal stump (5) is located;
- at least one auxiliary device comprising a ferromagnetic element, arranged at the point where the other of either said distal stump (4) or said proximal stump (5) is located and configured to interact by electromagnetic attraction with said device (1) so as to allow said distal stump (4) and said proximal stump (5) to approach each other;
- at least two reinforcing membranes (21) associable with the outer wall of said esophagus (3) at the point where said proximal stump (5) and said distal stump (4) are located, respectively, said reinforcing membranes (21) are made at least partly of a biocompatible and absorbable material.

14. Kit according to claim 13, **characterized by** the fact that it comprises at least two devices (1) according to one or more of claims 1-12, said magnetic elements (7) having opposite polarity.

## Patentansprüche

1. Vorrichtung (1) zur Behandlung von Ösophagusatresien und -stenosen, umfassend mindestens einen rohrförmigen Körper (2) mit länglicher Form, der dazu bestimmt ist, bei Gebrauch in einen von einer Ösophagusatresie betroffenen Ösophagus (3) eingeführt zu werden, und der in einen distalen Stumpf (4) und einen proximalen Stumpf (5) unterteilt ist, wobei die Vorrichtung mindestens ein magnetisches Element (7) umfasst, das mit einem Ende des rohrförmigen Körpers (2) verbunden und dazu bestimmt ist, an der Stelle platziert zu werden, an der sich entweder der distale Stumpf (4) oder der proximale Stumpf (5) befindet,
**gekennzeichnet durch** ein Verbindungsmittel (12), das mindestens eine rohrförmige Führung (13) umfasst, die in einen Durchgangskanal (14) eingeführt ist, der an dem magnetischen Element (7) ausgebildet ist, wobei die rohrförmige Führung (13) aus dem magnetischen Element (7) freitragend herausragt und durch gegenseitige Verriegelung mit dem rohrförmigen Körper (2) verbunden ist.

2. Vorrichtung (1) nach Anspruch 1, **dadurch gekennzeichnet, dass** sie mindestens ein Aufnahmegehäuse (11) für das magnetische Element (7) umfasst, das durch eine Anordnung des Verbindungsmittels (12) dazwischen mit dem rohrförmigen Körper (2) verbunden ist.

3. Vorrichtung (1) nach Anspruch 1, **dadurch gekennzeichnet, dass** die rohrförmige Führung (13) ein aufgeweitetes Ende (22) aufweist, das ausgebildet ist, durch Verriegelung mit dem rohrförmigen Körper (2) verbunden zu werden.

4. Vorrichtung (1) nach Anspruch 2, **dadurch gekennzeichnet, dass** das Aufnahmegehäuse (11) mindestens eine ringförmige Nut (15) aufweist, die ausgebildet ist, mindestens einen um das Aufnahmegehäuse (11) gewickelten Nahtfaden (16) aufzunehmen.

5. Vorrichtung (1) nach Anspruch 2 oder 4, **dadurch gekennzeichnet, dass** sie Schiebemittel (17) umfasst, die mit dem Aufnahmegehäuse (11) verbunden und ausgebildet sind, ein Gleiten des magnetischen Elements (7) innerhalb des Ösophagus (3) zu ermöglichen.

6. Vorrichtung (1) nach Anspruch 2, 4 oder 5, **dadurch gekennzeichnet, dass** sie Ausstoßmittel (18) umfasst, die mit dem Aufnahmegehäuse (11) verbunden und ausgebildet sind, die Freigabe des magnetischen Elements (7) innerhalb des Ösophagus (3) zu ermöglichen.

7. Vorrichtung (1) nach Anspruch 5 oder Anspruch 6, sofern von Anspruch 5 abhängig, **dadurch gekennzeichnet, dass** die Schiebemittel (17) mindestens ein Schieberohr (17) umfassen und die Ausstoßmittel (18) mindestens ein Ausstoßrohr (18) umfassen, wobei das Schieberohr (17) und das Ausstoßrohr (18) konzentrisch sind und in gleitender Weise miteinander verbunden sind.

8. Vorrichtung (1) nach Anspruch 7, **dadurch gekennzeichnet, dass** das eine, Schieberohr (17) und/oder das Ausstoßrohr (18), durch eine lösbare Verriegelung mit dem Aufnahmegehäuse (11) verbunden ist und das andere, Ausstoßrohr (18) und/oder das Schieberohr (17), an dem Aufnahmegehäuse (11) anliegt.

9. Vorrichtung (1) nach Anspruch 7 oder 8, **dadurch gekennzeichnet, dass**
• das Ausstoßrohr (18) durch eine Verriegelung lösbar mit dem Aufnahmegehäuse (11) verbunden ist;
• das Schieberohr (17) an dem Aufnahmegehäuse (11) anliegt und zwischen dem Ausstoßrohr (18) und dem rohrförmigen Körper (2) positioniert ist;
wobei das Schieberohr (17) aufgrund einer darauf ausgeübten Kraft von außen das Aufnahmegehäuse (11) wegschiebt und die Kopplung des Aufnahmegehäuse (11) von dem Ausstoßrohr (18) löst.

10. Vorrichtung (1) nach einem oder mehreren der vorhergehenden Ansprüche, **dadurch gekennzeichnet, dass** das magnetische Element (7) zumindest teilweise aus Neodym besteht.

11. Vorrichtung (1) nach einem oder mehreren der vorhergehenden Ansprüche, **dadurch gekennzeichnet, dass** sie mindestens einen Führungskörper (9) umfasst, der in dem rohrförmigen Körper (2) untergebracht ist und an einem Ende mit dem magnetischen Element (7) verbunden ist.

12. Vorrichtung (1) nach einem oder mehreren der vorhergehenden Ansprüche, **dadurch gekennzeichnet, dass** der Führungskörper (9) Versteifungsmittel (10) umfasst, die sich im Inneren des Führungskörpers selbst erstrecken.

13. Ausrüstung zur Behandlung von Ösophagusatresien und -stenosen, **dadurch gekennzeichnet, dass** sie umfasst:
• mindestens eine Vorrichtung (1) nach einem oder mehreren der vorhergehenden Ansprüche, die geeignet ist, an der Stelle angeordnet zu werden, an der der eine distale Stumpf (4) oder proximale Stumpf (5) angeordnet ist;
• mindestens eine Hilfsvorrichtung, die ein ferromagnetisches Element umfasst, die an der Stelle angeordnet ist, an der der andere distale Stumpf (4) oder proximale Stumpf (5) angeordnet ist, und ausgebildet ist, durch elektromagnetische Anziehung mit der Vorrichtung (1) zusammenzuwirken, um eine Annäherung des distalen Stumpfs (4) und des proximalen Stumpfs (5) aneinander zu ermöglichen;
• mindestens zwei Verstärkungsmembranen (21), die mit der Außenwand des Ösophagus (3) an der Stelle verbindbar sind, an der der proximale Stumpf (5) bzw. der distale Stumpf (4) angeordnet ist, wobei die Verstärkungsmembranen (21) zumindest teilweise aus einem biokompatiblen und resorbierbaren Material bestehen.

14. Ausrüstung nach Anspruch 13, **dadurch gekennzeichnet, dass** sie mindestens zwei Vorrichtungen (1) nach einem oder mehreren der Ansprüche 1 bis 12 umfasst, wobei die magnetischen Elemente (7) entgegengesetzte Polaritäten aufweisen.

## Revendications

1. - Dispositif (1) pour le traitement d'atrésies et sténoses de l'œsophage, comprenant au moins un corps tubulaire (2), de forme allongée, destiné, lors de l'utilisation, à être introduit à l'intérieur d'un œsophage (3) affecté par une atrésie de l'œsophage et divisé en un moignon distal (4) et en un moignon proximal (5), le dispositif comprenant
au moins un élément magnétique (7) associé à une extrémité dudit corps tubulaire (2) et destiné à être placé à l'endroit où est situé soit ledit moignon distal (4), soit ledit moignon proximal (5),
**caractérisé par** un moyen de jonction (12) comprenant au moins un conduit tubulaire (13) introduit à l'intérieur d'un canal traversant (14) réalisé sur ledit élément magnétique (7), ledit conduit tubulaire (13) étant en saillie en porte-à-faux à partir dudit élément magnétique (7) et couplé par verrouillage réciproque audit corps tubulaire (2).

2. - Dispositif (1) selon la revendication 1, **caractérisé par le fait qu'**il comprend au moins une enveloppe de confinement (11) dudit élément magnétique (7) associée audit corps tubulaire (2) par interposition du moyen de jonction (12).

3. - Dispositif (1) selon la revendication 1, **caractérisé par le fait que** ledit conduit tubulaire (13) présente une extrémité évasée (22) configurée pour être couplée par verrouillage réciproque audit corps tubulaire (2).

4. - Dispositif (1) selon la revendication 2, **caractérisé par le fait que** ladite enveloppe de confinement (11) comprend au moins une rainure annulaire (15) apte à loger au moins un fil de suture (16) enroulé autour de ladite enveloppe de confinement (11).

5. - Dispositif (1) selon la revendication 2 ou 4, **caractérisé par le fait qu'**il comprend des moyens de poussée (17) associés à ladite enveloppe de confinement (11) et configurés pour permettre audit élément magnétique (7) de coulisser à l'intérieur dudit œsophage (3).

6. - Dispositif (1) selon la revendication 2, 4 ou 5, **caractérisé par le fait qu'**il comprend des moyens d'éjection (18) associés à ladite enveloppe de confinement (11) et configurés pour permettre la libération dudit élément magnétique (7) à l'intérieur dudit œsophage (3).

7. - Dispositif (1) selon la revendication 5 ou la revendication 6 en dépendance de la revendication 5, **caractérisé par le fait que** lesdits moyens de poussée (17) comprennent au moins un tube pousseur (17) et lesdits moyens d'éjection (18) comprennent au moins un tube éjecteur (18), ledit tube pousseur (17) et ledit tube éjecteur (18) étant concentriques et associés l'un à l'autre d'une manière coulissante.

8. - Dispositif (1) selon la revendication 7, **caractérisé par le fait qu'**au moins l'un dudit tube pousseur (17) et dudit tube éjecteur (18) est couplé par verrouillage réciproque d'une manière amovible à ladite enveloppe de confinement (11) et que l'autre dudit tube pousseur (17) et dudit tube éjecteur (18) vient en butée contre ladite enveloppe de confinement (11).

9. - Dispositif (1) selon la revendication 7 ou 8, **caractérisé par le fait que** :
- ledit tube éjecteur (18) est couplé par verrouillage réciproque d'une manière amovible à ladite enveloppe de confinement (11) ;
- ledit tube pousseur (17) vient en butée contre ladite enveloppe de confinement (11) et est positionné entre ledit tube éjecteur (18) et ledit corps tubulaire (2) ;
ledit tube pousseur (17), sous l'effet d'une force externe exercée sur celui-ci, repousse ladite enveloppe de confinement (11) et libère le couplage de ladite enveloppe de confinement (11) vis-à-vis dudit tube éjecteur (18).

10. - Dispositif (1) selon l'une ou plusieurs des revendications précédentes, **caractérisé par le fait que** ledit élément magnétique (7) est constitué au moins en partie de néodyme.

11. - Dispositif (1) selon l'une ou plusieurs des revendications précédentes, **caractérisé par le fait qu'**il comprend au moins un corps de guidage (9) reçu dans ledit corps tubulaire (2) et associé, à une extrémité, audit élément magnétique (7).

12. - Dispositif (1) selon l'une ou plusieurs des revendications précédentes, **caractérisé par le fait que** ledit corps de guidage (9) comprend des moyens de raidissement (10) s'étendant intérieurement audit corps de guidage lui-même.

13. - Kit pour le traitement d'atrésies et sténoses de l'œsophage, **caractérisé par le fait qu'**il comprend :
- au moins un dispositif (1) selon l'une ou plusieurs des revendications précédentes approprié pour être disposé à l'endroit où est situé soit ledit moignon distal (4), soit ledit moignon proximal (5) ;
- au moins un dispositif auxiliaire comprenant un élément ferromagnétique, disposé à l'endroit où est situé l'autre dudit moignon distal (4) et dudit moignon proximal (5) et configuré pour interagir par attraction électromagnétique avec ledit dispositif (1) de façon à permettre audit moignon distal (4) et audit moignon proximal (5) de s'approcher l'un de l'autre;
- au moins deux membranes de renforcement (21) aptes à être associées à la paroi externe dudit œsophage (3) à l'endroit où sont situés respectivement ledit moignon proximal (5) et ledit moignon distal (4), lesdites membranes de renforcement (21) étant constituées au moins en partie d'un matériau biocompatible et résorbable.

14. - Kit selon la revendication 13, **caractérisé par le fait qu'**il comprend au moins deux dispositifs (1) selon l'une ou plusieurs des revendications 1 à 12, lesdits éléments magnétiques (7) ayant des polarités opposées.
